(19) Europäisches Patentamt
European Patent Office
Office européen des brevets

(11) Veröffentlichungsnummer: **0 243 369 B1**

(12) **EUROPÄISCHE PATENTSCHRIFT**

(45) Veröffentlichungstag der Patentschrift: **18.03.92**

(51) Int. Cl.5: **A61K 31/557**, C08B 37/16

(21) Anmeldenummer: **86901042.1**

(22) Anmeldetag: **04.02.86**

(86) Internationale Anmeldenummer:
**PCT/DE86/00039**

(87) Internationale Veröffentlichungsnummer:
**WO 86/04504 (14.08.86 86/18)**

Die Akte enthält technische Angaben, die nach dem Eingang der Anmeldung eingereicht wurden und die nicht in dieser Patentschrift enthalten sind.

# (54) 9-HALOGENPROSTAGLANDIN-CLATHRATE UND IHRE VERWENDUNG ALS ARZNEIMITTEL.

(30) Priorität: **04.02.85 DE 3504044**

(43) Veröffentlichungstag der Anmeldung:
**04.11.87 Patentblatt 87/45**

(45) Bekanntmachung des Hinweises auf die Patenterteilung:
**18.03.92 Patentblatt 92/12**

(84) Benannte Vertragsstaaten:
**AT BE CH DE FR GB IT LI LU NL SE**

(56) Entgegenhaltungen:
**EP-A- 0 030 377**
**EP-A- 0 069 696**
**DE-A- 2 128 674**

(73) Patentinhaber: **SCHERING AKTIENGESELLSCHAFT Berlin und Bergkamen**
**Müllerstrasse 170/178 Postfach 65 03 11**
**W-1000 Berlin 65(DE)**

(72) Erfinder: **RADÜCHEL, Bernd**
**Gollanczstrasse 132**
**W-1000 Berlin 28(DE)**
Erfinder: **SKUBALLA, Werner**
**Olwenstrasse 25**
**W-1000 Berlin 28(DE)**
Erfinder: **LOGE, Olaf**
**Bekassinenweg 37**
**W-1000 Berlin 27(DE)**
Erfinder: **TACK, Johann-Wilhelm**
**Tharsanderweg 42**
**W-1000 Berlin 20(DE)**

EP 0 243 369 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der vorliegenden Erfindung sind β-Cyclodextrin-Clathrate von 9-Halogenprostaglandinanaloga und diese enthaltende Mittel.

9-Chlor- oder 9-Fluorprostaglandinanaloga sind pharmakologisch und medizinisch wertvolle Wirkstoffe, deren Herstellung und Anwendung in DE-A-29 50 027 und 31 26 924 beschrieben sind. Diese Substanzen besitzen gegenüber den entsprechenden natürlichen Prostaglandinen bei ähnlichem Wirkungsspektrum eine wesentlich verbesserte Spezifität und vor allem wesentlich längere Wirkung.

Die in den erwähnten Offenlegungsschriften beschriebenen 9-Chlor- bzw. 9-Fluorprostaglandine liegen oft nicht kristallin vor, wodurch ihrer pharmazeutischen Anwendung Grenzen gesetzt sind. Hinzu kommt noch eine begrenzte Wasserlöslichkeit und Lösungsgeschwindigkeit.

Es wurde nun gefunden, daß Einschlußverbindungen dieser 9-Chlor- und 9-Fluorprostaglandine mit β-Cyclodextrinen die genannten Nachteile nicht besitzen, d.h. ihre Wasserlöslichkeit wird verbessert, die Lösungsgeschwindigkeit wird gesteigert und die Einschlußverbindungen liegen in kristalliner Form vor. Außerdem wird ihre Stabilität beispielsweise gegenüber Wärme, Licht und Sauerstoff erhöht und ihre galenische Zubereitung (Herstellen von Lösungen oder Tabletten) erleichtert.

Die Erfindung betrifft β-Cyclodextrin-Clathrate von Prostaglandinen der allgemeinen Formel I

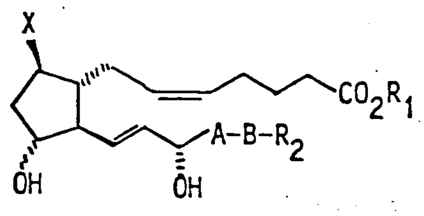

worin

| | |
|---|---|
| $R_1$ | ein Wasserstoffatom oder einen geradkettigen oder verzweigten Alkylrest mit bis zu 10 C-Atomen, |
| $R_2$ | ein geradkettiger oder verzweigter Alkylrest mit bis zu 10 C-Atomen, eine Cycloalkylgruppe mit 3-10 C-Atomen im Ring oder eine Phenyl-Gruppe die durch Halogen, $C_{1-4}$-Alkoxy, $C_{1-4}$-Alkyl, Trifluormethyl oder Halogen substituiertes $C_{1-4}$-Alkyl substituiert sein kann, |
| A und B | gemeinsam eine direkte Bindung oder |
| A | eine gerad- oder verzweigtkettige Alkylengruppe mit bis zu 10 C-Atomen und |
| B | ein Sauerstoffatom, eine direkte Bindung oder eine -C≡C-Bindung und |
| X | ein Chlor- oder Fluoratom bedeuten. |

Als gerad- oder verzweigtkettige Alkylgruppen $R_1$ sind beispielsweise Methyl, Äthyl, Propyl, Isopropyl, Butyl, Isobutyl, sek.-Butyl, tert.-Butyl, Pentyl, Hexyl, Decyl gemeint.

Als Alkylgruppen $R_2$ kommen gerad- und verzweigtkettige gesättigte und ungesättigte Alkylreste, vorzugsweise gesättigte, mit bis zu 10 C-Atomen infrage. Beispielsweise genannt seien Methyl, Äthyl, Propyl, Butyl, Isobutyl, Pentyl, Isobutenyl, Octyl oder 1,1-Dimethylpentyl.

Die Cycloalkylgruppe $R_2$ kann im Ring 3-10, vorzugsweise 5 oder 6 C-Atome enthalten. Beispielsweise genannt seien Cylcopropyl, Cyclobutyl, Cyclopentyl, Cyclohexyl.

Die Phenylgruppe $R_2$ kann gegebenenfalls durch Halogen, $C_{1-4}$-Alkoxy, $C_{1-4}$-Alkyl, Trifluormethyl oder halogen-substituiertes $C_{1-4}$-Alkyl substituiert sein.

Als Alkylengruppe A kommen geradkettige oder verzweigte, gesättigte und ungesättigte Alkylenreste, vorzugsweise gesättigte mit bis zu 10 C-Atomen, infrage. Beispielsweise seien genannt Methylen, Äthylen, Dimethylmethylen, Athyläthylen, Trimethylen, Tetramethylen.

$R_2$, A und B können so gewählt werden, daß die 16-Position durch ein oder zwei Alkylgruppen substituiert ist, z.B. Methyl oder Äthyl, vorzugsweise Methyl.

Zur Herstellung der erfindungsgemäßen Clathrate werden die Verbindungen der allgemeinen Formel I in einem pharmakologisch unbedenklichen Alkohol, vorzugsweise Ethanol, aufgelöst und zu wäßrigen Lösungen von β-Cyclodextrin, bei 60°C zugesetzt. Nach dem Abkühlen kristallisieren die entsprechenden Clathrate aus und können durch Absaugen und Trocknen als feste, frei fließende Kristalle isoliert werden, wobei das Molverhaltnis Cyclodextrin/Prostaglandin 1 bis 25:1, bevorzugt bei 3 bis 15:1 liegt. Die entsprechend dieser Erfindung hergestellten Clathrate sind wertvolle Pharmaka. Sie wirken stark magensäuresekretionshemmend und schützen die Magenschleimhaut gegen verschiedene schädliche Einflüsse wie beispielsweise Äthanol, Salzsäure, heißes Wasser und Streß sowie gegen entzündungshemmende Stoffe wie

Aspirin® oder Indometacin.

Einige der erfindungsgemäßen Clathrate sind hervorragend geeignet zur Behandlung der allergischen und vasomotorischen Rhinitis, da sie eine rasche Abschwellung der Nasenschleimhaut bewirken.

Einige der erfindungsgemäßen Clathrate sind außerdem geeignet, nach enteraler oder parenteraler Applikation eine Menstruation zu induzieren oder eine Schwangerschaft zu unterbrechen.

Sie eignen sich ferner zur Synchronisation des Sexualzyklus weiblicher Säugetiere wie Kaninchen, Rinder, Pferde und Schweine. Ferner eignen sie sich zur Cervixdilatation als Vorbereitung für diagnostische oder therapeutische Eingriffe.

Die erfindungsgemäßen Clathrate können in flüssigen oder festen galenischen Formulierungen verwendet werden, wobei die Formulierungen enteral, parenteral, vaginal oder rektal verabreicht werden können.

Zur Herstellung von Tabletten wird das Prostaglandin-Cyclodextrin-Clathrat mit Trägersubstanzen und Hilfsstoffen wie Laktose, Maisstärke, Polyvinylpyrrolidon und Magnesiumstearat vermischt.

Zur Herstellung von Lösungen für die enterale und parenterale Anwendung werden die wäßrigen Cyclodextrin-Clathrat-Lösungen zusammen mit Laktose lyophilisiert. Anschließend können die Lyophilisate mit physiologischer Kochsalzlösung auf die gewünschte Konzentration gebracht werden.

Die Erfindung umfaßt daher pharmazeutische Präparate und Formulierungen, die als Wirkstoff ein Cyclodextrin-Clathrat eines 9-Chlor- oder 9-Fluor-prostaglandinanalogons enthalten.

Die Erfindung wird durch folgende Beispiele erläutert:

## Beispiel 1

Man löst 4,90 g β-Cyclodextrin in 35 ml Wasser bei 80°C, kühlt auf 60°C ab und tropft eine Lösung von 175 mg (5Z,13E)-(9R,11R,15R)-9-Chlor-11,15-dihydroxy-16,16-dimethyl-5,13-prostadiensäure in 2.6 ml Äthanol innerhalb von 15 Minuten zu. Man rührt weitere 1.5 Std. bei 60°C und läßt dann über Nacht unter Rühren abkühlen. Der ausgefallene Feststoff wird abgesaugt, mit 25 ml einer Mischung aus Wasser-Äthanol (1:1) gewaschen und 6 Std. bei 40°Cund 13,3 Pa (0.1 Torr) über Phosphorpentoxid getrocknet. Man erhält 2,88 g frei fließende Kristalle des β-Cyclodextrin-Clathrats des o.a. 9-Chlor-prostaglandinanalogons.

Der Gehalt an 9-Chlor-prostaglandinanalogon im Clathrat wurde durch Hochdruckflüssigkeitschromatographie bestimmt und betrug 5,2 %.

## Beispiel 2

Zu einer Lösung von 1.225 g β-Cyclodextrin in 8,75 ml Wasser tropft man bei 60°C eine Lösung von 42 mg (5Z,13E)-(9R,11R,15R)-9-Fluor-11,15-dihydroxy-16-phenoxy-17,18,9,20-tetranor-5,13-prostadiensäure in 0,6 ml Äthanol, rührt 2 Std. bei 60°C und läßt dann abkühlen. Nach 24 Std. saugt man die Kristalle ab, wäscht mit 6 ml Wasser Äthanol (1:1) nach und trocknet bei 40°C 6 h bei 13,3 Pa (o.1 Torr) über Phosphorpentoxid. Man erhält 0,68 g frei fließende Kristalle des β-Cyclodextrin-Clathrats des o.a. 9-Fluorprostaglandinanalogons.

Der Gehalt an 9-Fluor-prostaglandinanalogon im Clathrat wurde durch Hochdruckflüssigkeitschromatographie bestimmt und betrug 5.1 %.

Verfährt man wie in den Beispielen 1 und 2 beschrieben, so lassen sich die β-Cyclodextrin-Clath rate der folgenden Analoga herstellen:

(5Z,13E)-(9R,11R,15R)-9-Chlor-11,15-dihydroxy-16-phenoxy-17,18,19,20-tetranor-5,13-prostadiensäure
(5Z,13E)-(9R,11R,15R)-9-Fluor-11,15-dihydroxy-16,16-dimethyl-5,13-prostadiensäure
(5Z,13E)-(9R,11R,15R)-9-Chlor-11,15-dihydroxy-16,16,20-trimethyl-18,18,19,19-tetradehydro-5,13-prostadiensäure.

## Beispiel 3

Das β-Cyclodextrin-Clathrat von (5Z,13E)-(9R,11R,15R)-9-Chlor-11,15-dihydroxy-16,16-dimethyl-5,13-prostadiensäure (Wirkstoffgehalt: 3.5 %) wird portionsweise mit Laktose angerieben und Polyvinylpyrrolidon 25 000 und Maisstärke Zugemischt. Die Mischung wird mit Aqua bidest. granuliert, gesiebt und getrocknet. Magnesiumstearat wird zugemischt und die erhaltene Preßmasse auf einer geeigneten Tablettenpresse zu Tabletten mit 6 mm Durchmesser verpreßt.

Zusammensetzung einer Tablette:

| | |
|---|---|
| ß-Cyclodextrin-Clathrat (s.o.) | 2.86 mg |
| Laktose | 50.44 mg |
| Maisstärke | 24.00 mg |
| Polyvinylpyrrolidon 25 000 | 2.40 mg |
| Magnesium-Stearat | 0.30 mg |
| | 80.00 mg |

Man erhält Tabletten zu 80 mg mit einem Gehalt von 100 µg (5Z,13E)-(9R,11R,15R)-9-Chlor-11,15-dihydroxy-16,16-dimethyl-5,13-prostadiensäure.

Beispiel 4

Das β-Cyclodextrin-Clathrat von (5Z,13E)-(9R,11R,15R)-9-Chlor-11,15-dihydroxy-16,16-dimethyl-5,13-prostadiensäure (Wirkstoffgehalt: 5.2 %) wird zusammen mit Laktose in Aqua bidest. gelöst, über ein geeignetes Filter in ein Multivial filtriert und bei 20 ° C eingefroren. Anschließend wird unter Vakuum 24 h gefriergetrocknet.

Zusammensetzung einer Einheit:

| | |
|---|---|
| β-Cyclodextrin-Clathrat (s.o.) | 1.94 mg |
| Laktose | 10.00 mg |
| Aqua bidest. | ad 2000.00 mg |

Man erhält auf diese Weise Formulierungen, die 100 µg (5Z,13E)-(9R,11R,15R)-9-Chlor-11,15-dihydroxy-16,16-dimethyl-5,13-prostadiensäure pro Multivial enthalten.

## Patentansprüche

**1.** Pharmazeutische Präparate, dadurch gekennzeichnet, daß sie als wirksame Komponente ein β-Cyclodextrin-Clathrat eines Prostaglandins der allgemeinen Formel I

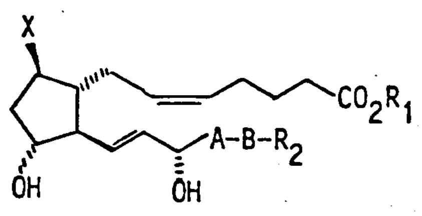

worin

| | |
|---|---|
| $R_1$ | ein Wasserstoffatom oder einen geradkettigen oder verzweigten Alkylrest mit bis zu 10 C-Atomen, |
| $R_2$ | ein geradkettiger oder verzweigter Alkylrest mit bis zu 10 C-Atomen, eine Cycloalkylgruppe mit 3-10 C-Atomen im Ring oder eine Phenyl-Gruppe die durch Halogen, $C_{1-4}$-Alkoxy, $C_{1-4}$-Alkyl, Trifluormethyl oder Halogen substituiertes $C_{1-4}$-Alkyl substituiert sein kann, |
| A und B | gemeinsam eine direkte Bindung oder |
| A | eine gerad- oder verzweigtkettige Alkylengruppe mit bis zu 10 C-Atomen und |
| B | ein Sauerstoffatom, eine direkte Bindung oder eine -C≡C-Bindung und |

X ein Chlor- oder Fluoratom bedeuten, zusammen mit pharmazeutisch unbedenklichen Trägersubstanzen und Hilfsstoffen enthalten.

**2.** Präparat nach Anspruch 1, dadurch gekennzeichnet, daß es ein (5Z,13E)-(9R,11R,15R)-9-Chlor-11,15-dihydroxy-16,16-dimethyl-5,13-prostadiensäure-β-Cyclodextrin-Clathrat enthält.

**3.** Präparat nach Anspruch 1, dadurch gekennzeichnet, daß es ein (5Z,13E)-(9R,11R,15R)-9-Fluor-11,15-dihydroxy-16-phenoxy-17,18,19,20-tetranor-5,13-prostadiensäure-β-Cyclodextrin-Clathrat enthält.

**4.** Präparat nach Anspruch 1, dadurch gekennzeichnet, daß es ein (5Z,13E)-(9R,11R,15R)-9-Chlor-11,15-dihydroxy-16-phenoxy-17,18,19,20-tetranor-5,13-prostadiensäure-β-Cyclodextrin-Clathrat enthält.

**5.** Präparat nach Anspruch 1, dadurch gekennzeichnet, daß es ein (5Z,13E)-(9R,11R,15R)-9-Fluor-11,15-dihydroxy-16,16-dimethyl-5,13-prostadiensäure-β-Cyclodextrin-Clathrat enthält.

**6.** Präparat nach Anspruch 1, dadurch gekennzeichnet, daß es ein (5Z,13E)-(9R,11R,15R)-9-Chlor-11,15-dihydroxy-16,16, 20-trimethyl-18,18,19,19-tetradehydro-5,13-prostadiensäure-β-Cyclodextrin-Clathrat enthält.

**7.** Cyclodextrinclathrate gemäß Anspruch 1, dadurch gekennzeichnet, daß das Molverhältnis Cyclodextrin/Prostaglandin der Formel I bei 1 bis 25:1 liegt.

**8.** Cyclodextrinclathrat nach Anspruch 7, dadurch gekennzeichnet, daß das Molverhältnis bei 3 bis 15:1 liegt.

**Claims**

**1.** Pharmaceutical preparations, characterised in that they comprise as active component a β-cyclodextrin-clathrate of a prostaglandin of the general formula I

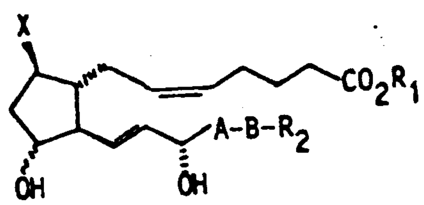

wherein

| | |
|---|---|
| $R_1$ | represents a hydrogen atom or a straight-chain or branched alkyl radical having up to 10 carbon atoms, |
| $R_2$ | represents a straight-chain or branched alkyl radical having up to 10 carbon atoms, a cycloalkyl group having from 3 to 10 carbon atoms in the ring or a phenyl group that may be substituted by halogen, $C_{1-4}$-alkoxy, $C_{1-4}$-alkyl, trifluoromethyl or by halo-substituted $C_{1-4}$-alkyl, |
| A and B | together represent a direct bond or |
| A | represents a straight-chain or branched alkylene group having up to 10 carbon atoms and |
| B | represents an oxygen atom, a direct bond or a -C≡C-bond and |
| X | represents a chlorine or fluorine atom, |

together with pharmaceutically acceptable carriers and auxiliaries.

**2.** Preparation according to claim 1, characterised in that it comprises a (5Z,13E)-(9R,11R,15R)-9-chloro-11,15-dihydroxy-16,16-dimethyl-5,13-prostadienoic acid β-cyclodextrin-clathrate.

**3.** Preparation according to claim 1, characterised in that it comprises a (5Z,13E)-(9R,11R,15R)-9-fluoro-11,15-dihydroxy-16-phenoxy-17,18,19,20-tetranor-5,13-prostadienoic acid β-cyclodextrin-clathrate.

4. Preparation according to claim 1, characterised in that it comprises a (5Z,13E)-(9R,11R,15R)-9-chloro-11,15-dihydroxy-16-phenoxy-17,18,19,20-tetranor-5,13-prostadienoic acid $\beta$-cyclodextrin-clathrate.

5. Preparation according to claim 1, characterised in that it comprises a (5Z,13E)-(9R,11R,15R)-9-fluoro-11,15-dihydroxy-16,16-dimethyl-5,13-prostadienoic acid $\beta$-cyclodextrin-clathrate.

6. Preparation according to claim 1, characterised in that it comprises a (5Z,13E)-(9R,11R,15R)-9-chloro-11,15-dihydroxy-16,16,20-trimethyl-18,18,19,19-tetradehydro-5,13-prostadienoic acid $\beta$-cyclodextrin-clathrate.

7. Cyclodextrin clathrates according to claim 1, characterised in that the molar ratio of cyclodextrin/prostaglandin of formula I is 1 to 25:1.

8. Cyclodextrin clathrate according to claim 7, characterised in that the molar ratio is 3 to 15:1.

**Revendications**

1. Préparations pharmaceutiques caractérisées en ce qu'elles comprennent comme composant actif un clathrate de $\beta$-cyclodextrine et d'une prostaglandine de formule générale I ci-dessous

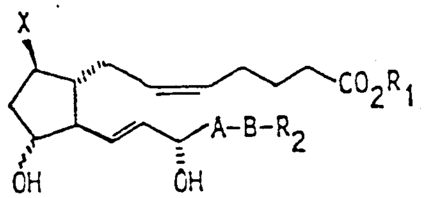

(I)

(dans laquelle :

| | |
|---|---|
| $R_1$ | désigne un atome hydrogène ou un alkyle à chaîne linéaire ou ramifiée pouvant avoir jusqu'à dix atomes de carbone, |
| $R_2$ | un alkyle à chaîne linéaire ou ramifiée pouvant avoir jusqu'à dix atomes de carbone, un cycloalkyle pouvant avoir de trois à dix atomes de carbone dans le cycle ou un phényle pouvant avoir comme substituants un halogène, un alcoxy $C_{1-4}$, un alkyle $C_{1-4}$, le groupe trifluorméthyle ou un halogénoalkyle $C_{1-4}$, |
| A et B | représentent ensemble une liaison directe ou bien |
| A | est un alkylène à chaîne liniaire ou ramifiée pouvant avoir jusqu'à dix atomes de carbone et |
| B | un atome d'oxygène, une liaison directe ou une liaison -C≡C-, et |
| X | représente un atome de chlore ou de fluor) avec des matières servant de support et des adjuvants sans inconvénient pour des usages pharmaceutiques. |

2. Préparation selon la revendication 1, caractérisée en ce qu'elle comprend un clathrate de $\beta$-cyclodextrine avec le (5Z,13E)-(9R,11R,15R)-9-chloro-11,15-dihydroxy-16,16-diméthyl-5,13-prostadiénoïque.

3. Préparation selon la revendication 1, caractérisée en ce qu'elle comprend un clathrate de $\beta$-cyclodextrine avec le (5Z,13E)-(9R,11R,15R)-9-fluoro-11,15-dihydroxy-16-phenoxy-17,18,19,20-tétranor-5,13-prostadiénoïque.

4. Préparation selon la revendication 1, caractérisée en ce qu'elle comprend un clathrate de $\beta$-cyclodextrine avec le (5Z,13E)-(9R,11R,15R)-9-chloro-11,15-dihydroxy-16-phenoxy-17,18,19,20-tétranor-5,13-prostadiénoïque.

5. Préparation selon la revendication 1, caractérisée en ce qu'elle comprend un clathrate de $\beta$-cyclodextrine avec le (5Z,13E)-(9R,11R,15R)-9-fluoro-11,15-dihydroxy-16-16-diméthyl-5,13-prostadiénoïque.

6. Préparation selon la revendication 1, caractérisée en ce qu'elle comprend un clathrate de $\beta$-cyclodextrine avec le (5Z,13E)-(9R,11R,15R)-9-chloro-11,15-dihydroxy-16,16,20-triméthyl-18,18,19,19-tétradéhydro-5,13-prostadiénoïque.

7. Clathrates de cyclodextrines selon la revendication 1, caractérisés en ce que le rapport molaire cyclodextrine/prostaglandine de formule I est compris entre 1 et 25:1.

8. Clathrates de cyclodextrines selon la revendication 7, caractérisé en ce que le rapport molaire se situe entre 3 et 15:1.